# EUROPEAN PATENT APPLICATION

(11) **EP 1 935 242 A1**
(43) Date of publication of application: **25.06.2008**
(21) Application number: 06026563.4
(22) Date of filing: 21.12.2006
(51) Int. Cl.: A01K 67/027, C12N 15/85, C12N 15/63

(54) **Transgenic animal model**

(71) Applicant: Philip Morris Products S.A., 2000 Neuchâtel (CH)
(72) Inventor: The designation of the inventor has not yet been filed
(74) Representative: Vossius & Partner

(57) **Abstract**

The present invention is related to a transgenic, non-human animal, particularly a transgenic rodent, but especially a transgenic mouse model which allows for the simultaneous, tissue-specific and temporally-controlled regulation of transgene expression and can be used as a tool to investigate the consecutive steps involved in initiation and progression of certain diseases such as cancer, but particularly lung cancer.

## Description

The present invention is related to a transgenic, non-human animal, particularly a transgenic rodent, but especially a transgenic mouse model which allows for the simultaneous, tissue-specific and temporally-controlled regulation of transgene expression.

Such model can be used as a tool to investigate the consecutive steps involved in initiation and progression of certain diseases such as cancer, but particularly lung cancer.

Transgenic mouse models have been an irreplaceable tool for the study of the molecular and physiological processes involved in certain human disease states, particularly those involved in oncogenicity. Whereas the gene disruption and gene replacement strategies used to develop a conventional transgenic mouse model are appropriate to create null mutants or gain-of-function mutants, neither is ideal to model diseases with polygenic etiologies and environmental influences such as, for example, cancer as an "acquired" disease. Such strategies create germline mutations. As a consequence, the potential to initiate a cascade of secondary responses during the earliest stages of development is substantial. These responses, even if phenotypically silent in the unstressed organism, may considerably after behavior to additional challenges in the adult mouse. Although this information, in itself, may be of interest, rendering a transgene silent during development and inducing its expression in the adult provides a potentially more suitable environment which is not complicated by potential developmental perturbations. Moreover, most cancers are sporadic, and carcinogenesis is often tissue or spatially specific. The conventional transgenic mouse carries constitutive transgenes in the complete animal, which creates a very different microenvironment than is found during initiation of sporadic cancers in which a few tumor cells are surrounded by many normal cells that may keep the incipient cancer cell in check by cell-cell contact or paracrine signaling.

Epidemiological data have clearly indicated that cigarette smoke is causally associated with the development of lung cancer. Past molecular biology studies have revealed the complex molecular alterations in lung cancer. It is known that perturbations of the integrity of integrated signaling networks, positively or negatively regulating various cellular processes to maintain homeostasis of the lung, lead to the progression of lung cancer. As with all types of cancer, transformation from a normal lung cell to a malignant lung cancer cell is the result of many combinations of events. Though carcinogenesis of cigarette smoke is believed to be a multistage process, there is little agreement as to which steps are truly required for cancer to develop. The cause and effect relationship between certain genetic or epigenetic aberrations and carcinogenesis is often unclear. In fact, "the carcinogenic mechanism(s) of tobacco smoking are still not well-understood". This is complicated by the fact that knowledge of the early events of cigarette smoke-induced carcinogenesis is lacking. Furthermore, both the cell type of origin of lung cancer(s) and the complex, even "paradoxical", role that the microenvironment plays in tumorigenicity are unknown.

Recent strategies for the development of transgenic mouse models for lung cancers have evolved from targeting transgenes in a constitutive mode to regulating the expression and ablation of genes in the lung in an inducible mode. This allows for the control of gene expression in a spatial- and tissue-specific manner, thereby providing a better platform for further investigation of the molecular basis of tumorigenesis (reviewed by Kwak et al., 2004). The most widely used systems in creating the conditional transgenic mouse are the Cre recombinase of *P1* bacteriophage and the Flp recombinase of *Saccharomyces cerevisiae* yeast, which behave quite similarly to each other. The recombinase enzyme Cre (or Flp) promotes recombination via recognition of a 34-bp asymmetric polynucleotide termed *loxP* (or frt). Depending on the relative orientation of the loxP sites, Cre may catalyze excision (same orientation) or insertion (opposite orientation) of the DNA segments lying between these sites. The approach to achieve inducible gene targeting involves two types of transgenic mouse lines. The first transgenic mouse line bears the target gene (or gene segment) flanked by two IoxP sites in the same orientation and positioned in such a way that it does not prevent normal gene activity (floxed gene). The second transgenic mouse line contains a transgene expressing Cre recombinase. When these two mouse lines are crossed, depending on the promoter/regutatory sequences present in the transgene, the floxed gene is deleted and a mutation is created in particular cells or tissues. It is important to note that two requirements must be fulfilled for conditional gene targeting with the Cre/loxP (or Flp/frt) system. Firstly, the floxed allele must be created in such a way that it is still functional, and secondly, targeting of Cre expression must be tightly controlled. Depending on the floxed DNA sequence, the Cre/IoxP system can also be used to create insertion. To this end, conditional transgenes can be introduced not only in a given type of cell but also at a given time in development.

These conditional models can be targeted to cancer, since it is a sporadic; i.e., acquired disease. Therefore, a genetic change can be introduced at any stage of the development of the mouse model. However, in addition to the fact that cancer is often an acquired disease and is subject to the sophisticated complication of the microenvironment, tobacco smoke carcinogenesis shows extra features. Epidemiological data showing that smokers, 15 to 25 years after smoke cessation, face almost the same low risk of developing lung cancer as the non-smoker strongly suggest a "reversibility" or dosage effect of cigarette smoke. Moreover, the insult imposed by cigarette smoke is often intermittent with respect to its mode of action. Several conditional transgenic mouse models for lung cancer have been developed by intranasally administering the adeno-cre virus, a recombinant adenovirus expressing Cre-recombinase (Jackson et al., 2001; Meuwissen et al., 2001). However, the suppression or activation of the targeted genes in this way is irreversible and becomes constitutive after the administration, which does not reflect the intermittent nature of cigarette smoke exposure or the observed reversibility of lung cancer in smokers following cessation. These points raise the question of relevance of the known conventional and conditional transgenic mice as proper disease models for cigarette smoke-induced lung tumorigenicity. In other words, at present there is no proper transgenic mouse model for studying cigarette smoke-induced lung cancer or other smoke-induced diseases.

In summary, an ideal or pertinent animal model for studying cigarette smoke-induced diseases should be able to capture: 1) the acquired nature of the diseases, 2) the complication of the microenvironment, 3) the intermittent characteristics of cigarette smoke exposure, and 4) the "reversible" nature of the carcinogenic process in smokers following cessation.

The present invention now provides such an animal model which possesses the above defined capabilities. In particular, the invention provides a transgenic, non-human animal comprising stably integrated into its genome a first and a second expression cassette, wherein said second expression cassette comprises a polynucleotide encoding an effector polypeptide under the control of a tissue-specific promoter, the expression of which is activated by the expression product of the first expression cassette, which expression product is encoded by a polynucleotide under the control of an inducible promoter.

According to all aspects and embodiments of the present invention, the preferred transgenic, non-human animal is a transgenic rodent, particularly a transgenic mouse.

In particular, the present invention provides a transgenic, non-human animal comprising stably integrated into its genome a first and a second expression cassette, wherein said second expression cassette comprises a polynucleotide encoding a second effector polypeptide under the control of a tissue-specific promoter and, optionally, a further nucleotide sequence, which blocks expression of the second effector polypeptide such that no expression of the effector nucleotide occurs from this promoter in the non-induced state, and wherein said first expression cassette comprises a polynucleotide encoding a first effector polypeptide under the control of an inducible promoter, which, upon induction by an inducing agent and expression, activates expression of the second effector polypeptide, for example by removing the block from the second expression cassette.

In a specific embodiment of the invention, a transgenic, non-human animal, is provided, wherein the first and second expression cassette are stably integrated in the genome of at least one somatic cell of said transgenic, non-human animal.

In another specific embodiment of the invention, a transgenic, non-human animal is provided, wherein the first and second expression cassette are stably integrated in the genome of at least one germline cell of said transgenic, non-human animal.

In still another specific embodiment of the invention, the second expression cassette comprises a further polynucleotide, which blocks expression of the second effector polynucleotide.

In another aspect of the invention, the blocking polynucleotide comprises a stop codon or a polyadenylation sequence or a reporter gene and is located in the expression cassette such that expression of the effector polypeptide is blocked, but especially between the promoter and the encoding polynucleotide.

In a specific embodiment of the invention, the polynucleotide encoding an effector polypeptide comprised in the first expression cassette under control of an inducible promoter is a recombinase encoding polynucleotide and the blocking sequence is flanked by short nucleotide sequences comprising recognition sites of the recombinase protein.

In particular, the effector polypeptide is a Cre recombinase or an Flp recombinase and the recombinase recognition sequences flanking the blocking polynucleotide, particularly the polyA sequence or stop codon or reporter gene are *loxP* and *frt* recognition sequences, respectively.

In a specific embodiment of the invention, the inducible promoter controlling the expression of the effector polypeptide in the first expression cassette is an on/off-type promoter which is strongly induced and provides essentially no background activity. In particular, induction of said promoter is dose dependent and compound/composition dependent and thus allows to fine tune gene expression levels and durations by modulating the dose and the nature of the inducing agent.

In a specific aspect of the invention an inducible promoter is provided which is strongly induced in the presence of environmental toxicants, for example, nicotine and polycyclic aromatic hydrocarbons (PAH) such as benzo(a)pyrene (BaP), and chlorinated dioxins and furans such as tetrachlorodibenzo-p-dioxin (TCDD), and beta-naphthoflavone (BNF), some of which are present in tobacco smoke, and the like, individually or in different combinations of one or more of said constituents.

In a specific embodiment of the invention, the inducible promoter controlling the expression of the effector polypeptide in the first expression cassette is a promoter controlling expression of a cytochrome P450 mono-oxygenase, but particularly expression of the cyp1A1 gene. Other inducible promoters may also be used within the scope of the present invention for controlling expression of the effector polypeptide such as, for example, without however being limited thereto, the promoter of stress responsive gene hsp70.1, promoters of metallotheonine genes and other cyp-type promoters.

In another aspect of the invention, the tissue-specific promoter controlling expression of the effector polypeptide comprised in the second expression cassette is a tissue specific promoter, particularly a lung tissue specific promoter, more particularly a promoter which controls expression of lung tissue-specific proteins, especially proteins that are specifically expressed in nonciliated bronchial epithelial cells (Clara cells) in respiratory and terminal bronchioles such as, for example, the Clara cell 10 protein, particularly the CC10 protein promoter.

In another embodiment of the invention, the lung tissue-specific promoter is a promoter which controls expression of lung tissue-specific proteins, more particularly proteins that are specifically expressed in alveolar epithelial cells such as, for example, the surfactant protein A/C. Such protein is a lung tissue-specific protein, which modulates a number of immune cell functions, including cell proliferation, cytokine production, the expression of cell surface markers, and the generation of oxidative activity.

In still another embodiment of the inventon, the lung tissue specific promoter is a promoter which controls expression of lung tissue-specific proteins in both type I and type II lung epithelial cells such as, for example, the RAIG1

Other lung- tissue-specific promoter that may also be used within the scope of the present invention, without however being limited thereto, include for example a promoter selected from the group consisting of thyroid transcription factor-2 (TTF1), promoter of aquaporin 5 and that of T1 alpha., and promoter of retinoic acid-induced gene 1 (RAIG1). Thyroid transcription factor-1 (TTF-1, product of the Nkx2.1 gene) is essential for branching morphogenesis of the lung and enhances expression of surfactant proteins by alveolar type II cells. T1 alpha is a differentiation gene of lung alveolar epithelial type I cells. Aquaporin-5 (AQP5) is a water channel protein expressed on the apical surface of alveolar epithelial type I cells. RAIG1 is a gene specifically expressed in lung epithelail cells of both type I and type II.

In a further aspect of the invention, the transgenic, non-human animal according to the present invention and described herein before comprises stably integrated in its genome a gene of interest (GOI) the expression of which is modulated by the second effector polypeptide.

In particular, a transgenic, non-human animal is provided comprising stably integrated into its genome a first and a second expression cassette, wherein said second expression cassette comprises a polynucleotide encoding an effector polypeptide under the control of a tissue-specific promoter, the expression of which is activated by the expression product of the first expression cassette which expression product is encoded by a polynucleotide under the control of an inducible promoter and wherein said transgenic, non-human animal comprises in its genome a gene of interest (GOI), which is integrated in the animal's genome in an active state and/or actively expressed from the genome, but becomes inactivated upon expression of the second effector polypeptide.

In another embodiment, the invention provides a transgenic, non-human animal comprising stably integrated into its genome a first and a second expression cassette, wherein said second expression cassette comprises a polynucleotide encoding a second effector polypeptide under the control of a tissue-specific promoter and, optionally, a further nucleotide sequence, which blocks expression of the second effector polypeptide such that no expression of the effector nucleotide occurs from this promoter in the non-induced state, and wherein said first expression cassette comprises a polynucleotide encoding a first effector polypeptide under the control of an inducible promoter, which, upon induction by an inducing agent or composition and expression of the first effector polypeptide, activates expression of the second effector polypeptide, for example by removing the block from the second expression cassette, and wherein said transgenic, non-human animal comprises in its genome a gene of interest (GOI), which is integrated into the animal's genome such that it is actively expressed from said genome in the non-induced state, but becomes inactivated upon expression of the second effector polypeptide.

The gene of interest may either be a native gene which is present in the genome of the transgenic, non-human animal, or, in the alternative, a transgene that has been introduced into the animal's genome artificially through recombinant DNA techniques,

The gene of interest may, for example, be a tumor suppressor gene such as the tumor suppressor gene k-Ras 12 (Jackson et al., 2001), Rb1 or Trp53 (Meuwissen et al., 2001), or any other gene that can be suitably used within the scope of the present invention, which gene is operably integrated into the genome of the transgenic, non-human animal and actively expressed from the genome in the non-induced state, but becomes blocked or inactivated upon expression of the second effector polypeptide.

In a specific embodiment of the invention, the second effector polypeptide is a recombinase and the gene of interest in the genome of the transgenic, non-human animal is flanked by short nucleotide sequences comprising recognition sites of a recombinase protein which do not interfere with the normal expression of the gene of interest.

In particular, the effector polypeptide is a Cre recombinase or an Flp recombinase and the recombinase recognition sequences flanking the gene of interest (GOI) are *IoxP* and *frt* recognition sequences, respectively.

In another aspect of the invention, the gene of interest (GOI) is integrated in the genome of the transgenic, non-human animal in an inactive state and/or not actively expressed from the genome.

In particular, a transgenic, non-human animal is provided comprising stably integrated into its genome a first and a second expression cassette, wherein said second expression cassette comprises a polynucleotide encoding a second effector polypeptide under the control of a tissue-specific promoter, the expression of which is activated by the expression product of the first expression cassette which expression product is encoded by a polynucleotide under the control of an inducible promoter and wherein said transgenic, non-human animal comprises in its genome a gene of interest (GOI), which is integrated in the animal's genome in an inactive state and/or is not actively expressed from the genome, but becomes activated upon expression of the second effector polypeptide.

In another embodiment, the invention provides a transgenic, non-human animal comprising stably integrated into its genome a first and a second expression cassette, wherein said second expression cassette comprises a polynucleotide encoding a second effector polypeptide under the control of a tissue-specific promoter and, optionally, a further nucleotide sequence, which blocks expression of the second effector polypeptide such that no expression of the effector nucleotide occurs from this promoter in the non-induced state, and wherein said first expression cassette comprises a polynucleotide encoding a first effector polypeptide under the control of an inducible promoter, which, upon induction by an inducing agent or composition and expression of a first effector polypeptide, activates expression of the second effector polypeptide, for example by removing the block from the second expression cassette, and wherein said transgenic, non-human animal comprises stably integrated in its genome a gene of interest (GOI), which is integrated in the animal's genome such that it is not actively expressed from said genome in the non-induced state, but becomes activated upon expression of the second effector polypeptide.

The blocking polynucleotide may comprise a stop codon or a polyadenylation sequence or a reporter gene and is located in the expression cassette such that expression of the effector polypeptide is blocked, but especially between the promoter and the encoding polynucleotide.

The gene of interest may either be a native gene which is present in the non-human animal's genome or, in the alternative, a transgene that has been introduced into the genome artificially through recombinant DNA techniques. The inactive gene of interest (GOI) is deblocked or activated upon expression of the second effector polypeptide such that expression of the gene of interest is caused.

The gene of interest may, for example, be a tumor susceptibility gene such as a tumor susceptibility gene selected from the group consisting of a lung adenoma susceptibility gene 1 (Las-1) and Kirstin rat sarcoma oncogene 2 (Kras2) or any other gene that can be suitably used within the scope of the present invention.

Further comprised by the present invention is a vector molecule comprising a first expression cassette and a second expression cassette, wherein said first expression cassette comprises a polynucleotide encoding a first effector polypeptide under the control of an inducible promoter, which, upon induction by an inducing agent or composition expresses a first effector polypeptide, and said second expression cassette comprises a polynucleotide encoding a second effector polypeptide under the control of a tissue-specific promoter, the expression of which is activated by the expression product of the first expression cassette.

In particular, the invention relates to a vector molecule, wherein the second expression cassette comprises a further polynucleotide, which blocks expression of the second effector polypeptide.

Also comprised is a vector molecule, wherein the inducible promoter controlling the expression of the effector polypeptide in the first expression cassette is an on/off-type promoter which is strongly induced and provides essentially no background activity and is dependent on the dose and the nature of the inducing compound or composition, particularly on cigarette smoke or at least one of its constituents, particularly a promoter controlling expression of a cytochrome P450 mono-oxygenase, but especially a promoter controlling expression of the cyp1A1 gene.

In a further embodiment, a vector molecule according to the present invention is provided, wherein the tissue-specific promoter is a lung tissue-specific promoter, particularly a promoter which controls expression of lung tissue-specific proteins, especially proteins that are specifically expressed in nonciliated bronchial epithelial cells (Clara cells) in respiratory and terminal bronchioles such as, for example, the Clara cell 10 protein, particularly the CC10 protein promoter. Alternatively, the vector molecule according to the present invention may comprise a lung tissue-specific promoter which controls expression of lung tissue-specific proteins that are specifically expressed in alveolar epithelial cells such as, for example, the surfactant protein A/C.

In a further aspect of the invention, the vector molecule according to the present invention comprises a first effector polypeptide, which is a recombinase, particularly a Cre recombinase or a Flp recombinase and/or a second effector polypeptide, which is a recombinase, particularly a Cre recombinase or a Flp recombinase.

Further provided is a vector molecule according to the invention, wherein the gene of interest is a tumor suppressor gene or a tumor susceptibility gene.

In yet another embodiment, the present invention provides a method of producing a transgenic non-human animal, particularly a transgenic rodent, but especially a transgenic mouse according to the present invention comprising transfecting a target animal with a vector molecule according to the invention and as described herein before.

In still another embodiment of the invention, a method is provided of evaluating the carcinogenic potential of an agent or a composition in a specific tissue of the animal when applied intermittently comprising: (i) contacting the transgenic, non-human animal according to the invention and as described herein before with the agent or composition to be evaluated; (ii) blocking or inactivating expression of a gene of interest thus causing genetic aberrations within the cells of a specific tissue of the animal, (iii) identifying and determining said genetic aberrations; (iv) comparing the number of genetically altered cells in a sample from the treated animal with the number of genetically altered cells in a sample from an untreated transgenic animal or transgenic animal treated with a control agent, wherein the difference in the number of transformed cells in the treated animal, relative to the number of transformed cells in the absence of treatment or treatment with a control agent, indicates the carcinogenic potential of the test compound.

Also comprised by the present invention is a method for evaluating the reversibility of the carcinogenic process induced by an agent or composition comprising: (i) contacting the transgenic, non-human animal according to the invention and as described herein before with the agent or composition to be evaluated intermittently according to a defined time schedule; (ii) blocking or inactivating expression of a gene of interest thus causing genetic aberrations within the cells of a specific tissue of the animal, (iii) identifying and determining said genetic aberrations; (iv) discontinuing contacting the transgenic animal with the agent or composition to be evaluated, comparing the number of genetically altered cells in a sample from the intermittently treated animal with the number of altered cells in a sample from a animal at a given time after treatment had been discontinued, and (v) determining reversibility of the carcinogenic process.

Many diseases which originate from some type of insult to a particular tissue or cell of the organism such as, for example, cigarette smoke-induced diseases, particularly cigarette smoke-induced cancer have the nature of "acquiredness". This type of disease is referred to as a sporadic disease as opposed to a familial disease. Therefore, the development of the disease is affected by the complication of the microenvironment, which can either stop, slow down, or speed up the disease. Moreover, in case of certain cigarette smoking-related diseases epidemiological evidence clearly illustrates that these diseases can be considered as being "reversible".

Therefore, the present invention provides an animal model which reflects both, the dose delivered and the intermittent mode of the exposure. In particular, the transgenic, non-human animal according to the present invention allows for a tissue-specific, particularly lung tissue-specific and temporally controlled expression of a gene of interest caused by the intermittent exposure of the model animal to a disease-inducing compound or composition, particularly by exposure to cigarette smoke followed by a transgene activation or inactivation of the gene of interest.

In a specific embodiment of the invention, a transgenic, non-human animal is provided wherein an expression cassette comprising a tissue-specific promoter, particularly a lung tissue-specific promoter such as, for example, a SP-A/C or CC10 promoter is combined with another expression cassette comprising an inducible promoter, particularly a promoter induced by environmental toxicants such as, for example, cigarette smoke or one of more of its constituents, particularly a cyp1A1 promoter, wherein both promoters drive the expression of an effector gene, particularly a recombinase gene such as, for example, a *cre* or an *flp* recombinase gene, which provides a feasible and powerful tool for investigating the consecutive steps involved in initiation and progression of certain diseases such as cancer, but particularly lung cancer.

A lung tissue-specific promoter that can be suitably used within the non-human animal model system according to the invention is the SP-A/C promoter or the CC10 promoter, respectively. Surfactant protein A/C is a lung tissue-specific protein, which modulates a number of immune cell functions, including cell proliferation, cytokine production, the expression of cell surface markers, and the generation of oxidative activity. It may also participate in the adaptive immune response. Due to its lung tissue-specificity, surfactant protein A/C promoter has been used in several studies to introduce genetic alterations in lung cells (Harrod et al., 1998; Wilmott et al., 1998). Clara cell 10 protein is the predominant product from nonciliated bronchial epithelial cells (Clara cells) in respiratory and terminal bronchioles in the lung. Since approximately 50-70% of epithelial cells in the trachea, bronchia, and bronchioles (pulmonary conducting airways) of mice are Clara cells, the Clara cell CC10 protein promoter has also been used to express transgenes in a lung tissue-specific manner in several studies (Temann et al., 1998; Fisher et al., 2001;).

An inducible promoter that can be suitably used within the model system according to the invention is the cyp1A1 promoter. The cyp1A1 gene is a gene from the cytochrome P450 mono-oxygenase system. The cytochrome P450 mono-oxygenase system represents a major defense against chemical challenge from the environment, constituting part of an adaptive response mounted by an organism following exposure to harmful agents, such as cigarette smoke. They also participate in a variety of essential "house-keeping" functions, such as biosynthesis of steroid hormones and fatty acid oxidation. Cytochrome P450s, however, are also able to catalyse the activation of certain compounds to toxic products.

These enzymes are highly regulated by chemical inducing agents. Of particular interest in this regard is the cytochrome P-450 cyp1A1 gene. This gene is not constitutively expressed but is highly inducible upon exposure to cigarette smoke and/or the constituents thereof and environmental toxicants such as, nicotine, polycyclic aromatic hydrocarbons (PAH), tetrachlorodibenzo-p-dioxin (TCDD), and beta-naphthoflavone (BNF), etc. A significant advantage of the cyp1A1 system is that there is essentially no background activity, and it provides the ability to fine tune gene expression levels and durations by modulating the dose and the nature of the inducing agent. Indeed several studies have shown that the cyp1A1 promoter in transgenic mice is an exquisitely sensitive on-off system for cell specific gene regulation (Campbell et al., 1996; Smith et al., 1995; Ryding et al., 2001). Not only is the expression of cyp1A1 induced by exposure to cigarette smoke, it is also induced by various environmental toxicants. This opens up the passibility of developing this type of conditional transgenic mice for toxicological testing and environmental monitoring as well.

In particular, the invention provides a transgenic, non-human animal comprising stably integrated into the mouse genome a first and a second expression cassette, wherein said second expression cassette comprises a polynucleotide encoding an effector polypeptide under the control of a tissue-specific promoter, particularly a lung tissue specific promoter such as, for example the Clara cell CC10 protein promoter or the surfactant protein A/C promoter or a promoter that controls expression of lung tissue specific proteins in both type I and type II lung epithelial cells such as, for example, the RAIG1, the expression of which is activated by the expression product of the first expression cassette which expression product is encoded by a polynucleotide under the control of an inducible promoter, particularly a promoter that is inducible by cigarette smoke or by one or more of its constituents such as, for example, the cyp'lA1 promoter.

In another embodiment, the invention provides a transgenic, non-human animal, particularly a mouse comprising stably integrated into the mouse genome a first and a second expression cassette, wherein said second expression cassette comprises a polynucleotide encoding an effector polypeptide, particularly a recombinase such as, for example, a Cre recombinase or a Flp recombinase, under the control of a tissue-specific promoter, particularly a lung tissue-specific promoter such as, for example the Clara cell CC10 protein promoter or the surfactant protein A/C promoter, the expression of which is activated by the expression product of the first expression cassette which expression product is an effector polypeptide, particularly a recombinase such as, for example, a Cre recombinase or a Flp recombinase, encoded by a polynucleotide under the control of an inducible promoter, particularly a promoter that is inducible by cigarette smoke or by one or more of its constituents such as, for example, the cyp1A1 promoter and a further polynucleotide, which blocks expression of the second effector polypeptide in the non-induced state, particularly a polynucleotide comprising a stop codon or a polyadenylation sequence or a reporter gene which is located in the expression cassette such that expression of the effector polypeptide is blocked, but especially between the promoter and the encoding polynucleotide and is flanked by recombinase recognition sequences such as, for example, *loxP* and *frt* recognition sequences.

In a further embodiment, the transgenic, non-human animal according to the present invention and as described herein before comprises in its genome a gene of interest (GOI), for example,a tumor suppressor gene such as the tumor suppressor gene k-Ras 12, Rb1, Trp53, the WT1 or *TSLC1* or a tumor suppressor gene located on the short arm of chromosome 3, in an area designated as 3p21.3 such as, for example the Ras association domain family 1 (RASSFI) including RASSFIA and RASSFIC, or any other gene that can be suitably used within the scope of the present invention, which is integrated in the animals genome such that it is actively expressed from the animals genome in the non-induced state, but becomes inactivated upon expression of the second effector polypeptide.

In a specific embodiment of the invention, the second effector polypeptide is a recombinase, particularly a Cre recombinase or a Flp recombinase, but especially a FLP recombinase and the gene of interest in the animal's genome is flanked by short polynucleotides comprising recognition sites of the recombinase protein which do not interfere with the normal expression of the gene of interest, particularly *loxP* or *frt* recognition sequences, but especially *frt* recognition sequences.

The dual promoter system according to the present invention comprising an inducible promoter, particularly a promoter inducible by cigarette smoke or one or more of its constituents and a tissue-specific promoter, particularly a lung tissue specific promoter, allows for control of the initiation of carcinogenesis in a tissue-specific and controlled manner, particularly in a lung tissue-specific and cigarette smoke controlled manner.

In a specific embodiment of the invention, a first mouse A, the transactivator mouse, is provided in which the sporadic expression of a recombinase, particularly a recombinase Cre is lung tissue-specific and regulated by a cigarette smoke-inducible promoter, particularly a cyp1A1 promoter. This mouse A is then crossed with a second mouse B comprising in its genome a gene of interest targeted with recombinase recognition sequences, particular a floxed targeted transgene such as floxed k-Ras 12 (Jackson et al., 2001) or floxed p53 and Rb (Meuwissen et al., 2001), wherein the gene is flanked by Cre recognition sequences.

The methods for producing mouse A and B used in this cross are well known in the art and described, for example, in Nagy et al., 2002: Manipulating the mouse embryos: A laboratory manual; Cold Spring Harbor Laboratory Press, 3rd edition.

The offspring of said cross which carries the dual promoter system and the floxed targeted genes can be used to study molecular mechanisms of cigarette smoke-induced diseases. Initiation of carcinogenesis in these mice can be controlled with respect to time by cigarette smoke exposure. Since recombinase Flp (and hence Cre as well) will only be sporadically or temporally induced by cigarette smoke, the genetic mutation or aberration will occur only in several proximal cells at each time of exposure and after cessation of the exposure gene expression may be restored, which reflects the intermittent exposure of cigarette smoke in smokers. Since surfactant protein A/ C or Clara cell 10 protein is only expressed in lung tissue, the expression of recombinase Cre and hence the genetic aberration is tissue-specifically controlled in the lung tissues. The transactivator mouse can also be used to generate transgenic mice in which gene expression is knocked down or down regulated with temporal and lung tissue specificity.

In one embodiment of the invention a method is therefore provided for evaluating the carcinogenic potential of an agent or a composition in a specific tissue upon intermittent exposure to said agent or composition comprising: (i) intermittently exposing a transgenic non-human animal, particularly a transgenic rodent, but especially a transgenic mouse according to the present invention and as described herein before to an agent or composition to be evaluated thus inducing sporadically or temporally an effector polypeptide in a tissue-specific manner; (ii) blocking or inactivating expression of a gene of interest thus causing genetic aberrations within the cells of a specific tissue of the animal, (iii) identifying and determining said genetic aberrations; (iv) comparing the number of genetically altered cells in a sample from the treated animal with the number of genetically altered cells in a sample from an untreated transgenic animal or transgenic animal treated with a control agent, wherein the difference in the number of transformed cells in the treated animal, relative to the number of transformed cells in the absence of treatment or treatment with a control agent, indicates the carcinogenic potential of the test compound.

In a specific aspect of the invention, a method is provided for evaluating the carcinogenic potential of cigarette smoke or of at least one constituent thereof in lung tissue upon intermittent exposure to cigarette smoke or to at least one constituent thereof comprising: (i) intermittently exposing a transgenic non-human animal, particularly a transgenic rodent, but especially a transgenic mouse according to the present invention and as described herein before to cigarette smoke or the at least one constituent thereof to be evaluated thus inducing sporadically or temporally an effector polypeptide in a lung tissue-specific manner; (ii) blocking or inactivating expression of a gene of interest thus causing genetic aberrations within the cells of a specific tissue of the animal, (iii) identifying and determining said genetic aberrations; (iv) comparing the number of genetically altered cells in a sample from the treated animal with the number of genetically altered cells in a sample from an untreated transgenic animal or transgenic animal treated with a control agent, wherein the difference in the number of transformed cells in the treated animal, relative to the number of transformed cells in the absence of treatment or treatment with a control agent, indicates the carcinogenic potential of cigarette smoke or of at least one constituent thereof.

In another aspect of the invention, a method is provided for evaluating the reversibility of the carcinogenic process induced by an agent or composition comprising: (i) intermittently exposing according to a defined time schedule the transgenic non-human animal, particularly a transgenic rodent, but especially a transgenic mouse according to the present invention and as described herein before to the agent or composition to be evaluated; (ii) blocking or inactivating expression of a gene of interest thus causing genetic aberrations within the cells of a specific tissue of the animal, (iii) identifying and determining said genetic aberrations; (iv) discontinuing exposure of the transgenic animal to the agent or composition to be evaluated, (iv) comparing the number of genetically altered cells in a sample from the intermittently treated test animal with the number of altered cells in a sample from an animal at a given time after treatment had been discontinued, and (iv) determining reversibility of the carcinogenic process.

In still another embodiment, the invention relates to a method for evaluating the reversibility of the carcinogenic process induced by cigarette smoke or at least one constituent thereof in lung tissue comprising: (i) intermittently exposing according to a defined time schedule the transgenic non-human animal, particularly the transgenic rodent, but especially the transgenic mouse according to the present invention and as described herein before to cigarette smoke or to at least one constituent thereof to be evaluated; (ii) blocking or inactivating expression of a gene of interest thus causing genetic aberrations within the cells of a specific tissue of the animal, (iii) identifying and determining said genetic aberrations; (iv) discontinuing exposure of the transgenic animal to cigarette smoke or at least one constituent thereof to be evaluated, (iv) comparing the number of genetically altered cells in a sample from the intermittently treated test animal with the number of altered cells in a sample from an animal at a given time after treatment had been discontinued, and (iv) determining reversibility of the carcinogenic process.

The transgenic non-human animal model according to the present invention can thus be used for investigate the significance of certain genetic or epigenetic aberrations in cigarette smoke-induced carcinogenesis or other diseases in a spatial- and temporal-specific manner.

### EXAMPLES

### Example 1: Construction of expression cassette 1 for inducible expression of Flp

For the inducible expression of Flp, a vector is constructed using standard rDNA techniques such as those described in Campbell et al, 1996 or Ryding et al, 2001. The flp transgene is operably linked with the inducible cyp1a1 promoter and the metallotheonine (MT-1) polyadenylation sequences such that the coding sequence of Flp recombinase is expressed under the control of the inducible promoter.

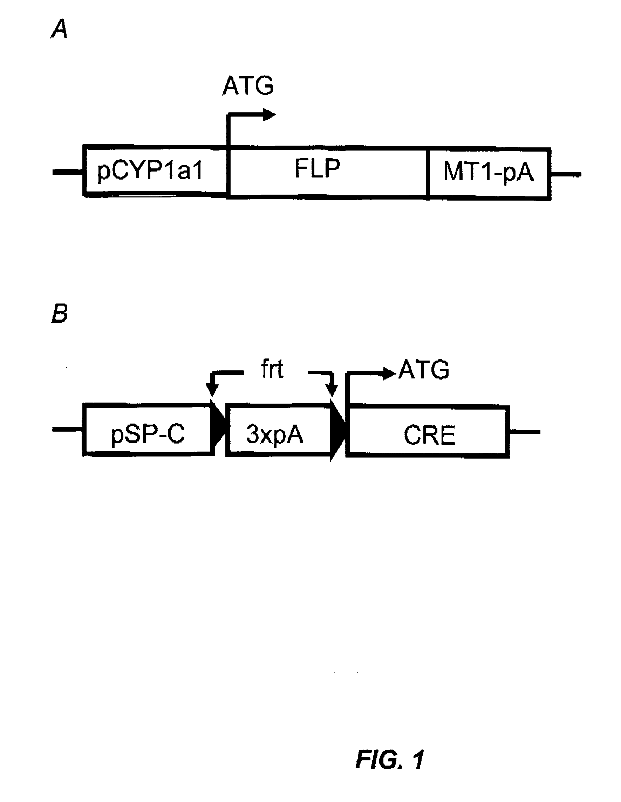

A schematic diagram of the vector construct is given in Figure 1.

### Example 2: Construction of expression cassette 2 for conditional expression of Cre

For the conditional expression of Cre in lung tissue a lung tissue-specittc promoter SP-C is fused to three MT-1 polyadenylation sequences which are flanked by Flp targeting sequences frt followed by a Cre recombinase coding sequence using standard rDNA techniques such as described, for example, in Harrod et al, 1998 or Wilmott et al, 1998 or Bertin et al, 2005.

A schematic diagram of the vector construct is given in Figure 1.

The pSP-C promoter in figure 1B can be the promoter of SP-A, that of Clara cell 10 protein, that of thyroid transcription factor 1 (TTF-1), that of T1 alpha, that of aquaporin 5, or that of RAIG-1 gene.

### Example 3: Transfection of expression cassette 1 and 2 into mouse A

Expression cassettes 1 and 2 are transfected into a first mouse A, the transactivator mouse, simultaneously or consecutively using standard transfection technology. Mouse A provides for sporadic expression of a recombinase, particularly a recombinase Cre in a lung tissue-specific manner and regulated by a cigarette smoke-inducible promoter, particularly a cyp1A1 promoter.

In this configuration, the Flp expression, which can be induced by cigarette smoke, results in the deletion of the three MT-1 polyadenylation sequences and thus the expression of Cre in lung tissue.

### Example 4: Transfection of a vector comprising a gene of interest (GOI) into mouse B,

A vector comprising the gene of interest such as k-Ras 12 (Jackson et al., 2001) or p53 and Rb (Meuwissen et al., 2001) is constructed as described for example in Kwak et al (2004) and transfected into a second mouse B using standard transfection technology to produce a mouse comprising in its genome a gene of interest targeted with recombinase recognition sequences, particular a floxed targeted transgene such as floxed k-Ras 12 or floxed p53 and Rb, wherein the gene is flanked by Cre recognition sequences.

### Example 5: Crossing of Mouse A X Mouse B

Mouse A is crossed with mouse B to produce offspring. The offspring of said cross carries the dual promoter system and the floxed targeted gene of interest and can be used to study molecular mechanisms of cigarette smoke-induced diseases. Initiation of carcinogenesis in these mice can be controlled with respect to time by cigarette smoke exposure. Since recombinase Flp (and hence Cre as well) will only be sporadically or temporally induced by cigarette smoke, the genetic mutation or aberration will occur only in several proximal cells at each time of exposure and after cessation of the exposure gene expression may be restored, which reflects the intermittent exposure of cigarette smoke in smokers. Since surfactant protein A/ C or Clara cell 10 protein is only expressed in lung tissue, the expression of recombinase Cre and hence the genetic aberration is tissue-specifically controlled in the lung tissues.

### Reference List

Bertin et al. 2005, Transgenic Research 14, 645-654
Campbell et al., 1996; Regulation of the CYP1A1 promoter in transgenic mice: an exquisitely sensitive on-off system for cell specific gene regulation. J Cell Sci. 109 (Pt 11): 2619-2625
Fisher et al., 2001, Induction and apoptotic regression of lung adenocarcinomas by regulation of a K-Ras transgene in the presence and absence of tumor suppressor genes, Genes Dev. 15:3249-3262
Harrod et al., 1998; Lung-specific expression of adenovirus E3-14.7K in transgenic mice attenuates adenoviral vector-mediated lung inflammation and enhances transgene expression. Hum Gene Ther. 9:1885-1898
Jackson et al., 2001; Analysis of lung tumor initiation and progression using conditional expression of oncogenic K-ras. Genes Dev. 15:3243-3243
Kwak et al., 2004; Geneticaly engineered mouse models for lung cancer, Annue Rev. Physiol. 66:647-663
Meuwissen et al., 2001; Mouse model for lung tumorigenesis through Cre/lox controlled sporadic activation of the K-Ras oncogene. Oncogene. 20:6551-6558
Ryding et al., 2001; Conditional transgenic technologies. J Endocrinol. 171:1-14
Smith et al., 1995; Cytochrome P450 1A1 promoter as a genetic switch for the regulatable and physiological expression of a plasma protein in transgenic mice. Proc Natl Acad Sci USA 92:11926-11930
Temann et al., 1998; Expression of interleukin 9 in the lungs of transgenic mice causes airway inflammation, mast cell hyperplasia, and bronchial hyperresponsiveness. J Exp Med 188:1307-1320
Wilmott et al., 1998; Generation of a transgenic mouse with lung-specific overexpression of the human interleukin-1 receptor antagonist protein. Am J Respir Cell Mol Biol. 18:429-434

## Claims

1. A transgenic animal comprising stably integrated into the animal genome a first and a second expression cassette, wherein said second expression cassette comprises a polynucleotide encoding a second effector polypeptide under the control of a tissue-specific promoter, the expression of which is activated by the expression product of the first expression cassette which expression product is encoded by a polynucleotide under the control of an inducible promoter.

2. A transgenic animal according to claim 1 comprising stably integrated into the animal genome a first and a second expression cassette, wherein said second expression cassette comprises a polynucleotide encoding a second effector polypeptide under the control of a tissue-specific promoter and, optionally, a further nucleotide sequence, which blocks expression of the second effector polypeptide such that no expression of the effector nucleotide occurs from this promoter in the non-induced state, and wherein said first expression cassette comprises a polynucleotide encoding a first effector polypeptide under the control of an inducible promoter, which, upon induction by an inducing agent or composition and expression of the first effector polypeptide, removes the block from the second expression cassette and activates expression of the second effector polypeptide.

3. A transgenic animal according to claim 1 comprising stably integrated into the animal genome a first and a second expression cassette, wherein said second expression cassette comprises a polynucleotide encoding an effector polypeptide under the control of a tissue-specific promoter, the expression of which is activated by the expression product of the first expression cassette which expression product is encoded by a polynucleotide under the control of an inducible promoter and wherein said transgenic animal comprises in its genome a gene of interest (GOI), which is integrated in the animal genome in an active state and/or actively expressed from the genome, but becomes inactivated upon expression of the second effector polypeptide.

4. A transgenic animal according to claim 1 comprising stably integrated into the animal genome a first and a second expression cassette, wherein said second expression cassette comprises a polynucleotide encoding a second effector polypeptide under the control of a tissue-specific promoter and, optionally, a further nucleotide sequence, which blocks expression of the second effector polypeptide such that no expression of the effector nucleotide occurs from this promoter in the non-induced state, and wherein said first expression cassette comprises a polynucleotide encoding a first effector polypeptide under the control of an inducible promoter, which, upon induction by an inducing agent or composition and expression of the first effector polypeptide, removes the block from the second expression cassette and activates expression of the second effector polypeptide, and wherein said transgenic animal comprises in its genome a gene of interest (GOI), which is integrated in the animal genome such that it is actively expressed from the animal genome in the non-induced state, but becomes inactivated upon expression of the second effector polypeptide.

5. A transgenic animal according to any one of claims 1 to 5, wherein the inducible promoter controlling the expression of the effector polypeptide in the first expression cassette is an on/off-type promoter which is strongly induced and provides essentially no background activity, particularly a promoter controlling expression of a cytochrome P450 mono-oxygenase or a promoter controlling expression of the cyp1A1 gene, which promoter is dependent on the dose and the nature of the inducing compound or composition, particularly a composition comprising cigarette smoke or at least one of its constituents such as, for example a compound selected from the group Nicotine, polycyclic aromatic hydrocarbons (PAH) such as benzo(a)pyrene (BaP), and chlorinated dioxins and furans such as tetrachlorodibenzo-p-dioxin (TCDD), and beta-naphthoflavone (BNF), etc, individually or in different combinations of one or more of said constituents.

6. A transgenic animal according to any of the preceding claims, wherein the tissue specific promoter is a lung tissue-specific promoter, particularly a promoter which controls expression of lung tissue-specific proteins, especially proteins that are specifically expressed
a) in nonciliated bronchial epithelial cells (Clara cells) in respiratory and terminal bronchioles such as, for example, the Clara cell 10 protein, particularly the CC10 protein promoter;
b) in alveolar epithelial cells such as, for example, the surfactant protein A/C;
c) in both type I and type II lung epithelial cells such as, for example, the RAIG1.

7. A transgenic animal according to any of the preceding claims, wherein the first and the second effector polypeptide are recombinases, particularly a Cre recombinase or an Flp recombinase.

8. A vector molecule comprising a first and a second expression cassette, wherein said first expression cassette comprises a polynucleotide encoding a first effector polypeptide under the control of an inducible promoter, which, upon induction by an inducing agent or composition expresses a first effector polypeptide, and said second expression cassette comprises a polynucleotide encoding a second effector polypeptide under the control of a tissue-specific promoter and, optionally, a further nucleotide sequence, which blocks expression of the second effector polypeptide such that no expression of the effector nucleotide occurs from this promoter in the non-induced state, the expression of which is activated by the expression product of the first expression cassette.

9. A vector molecule according to claims 9, wherein the
a) inducible promoter controlling the expression of the effector polypeptide in the first expression cassette is an on/off-type promoter which is strongly induced and provides essentially no background activity, particularly a promoter controlling expression of a cytochrome P450 mono-oxygenase or a promoter controlling expression of the cyp1A1 gene, which promoter is dependent on the dose and the nature of the inducing compound or composition, particularly a composition comprising cigarette smoke or at least one of its constituents such as, for example a compound selected from the group Nicotine, polycyclic aromatic hydrocarbons (PAH) such as benzo(a)pyrene (BaP), and chlorinated dioxins and furans such as tetrachlorodibenzo-p-dioxin (TCDD), and beta-naphthoflavone (BNF), etc, individually or in different combinations of one or more of said constituents; and
b) the tissue specific promoter is a lung tissue-specific promoter, particularly a promoter which controls expression of lung tissue-specific proteins, especially proteins that are specifically expressed
i. in nonciliated bronchial epithelial cells (Clara cells) in respiratory and terminal bronchioles such as, for example, the Clara cell 10 protein, particularly the CC10 protein promoter;
ii. in alveolar epithelial cells such as, for example, the surfactant protein A/C;
*iii.* in both type I and type II lung epithelial cells such as, for example, the RAIG1.

10. A method of producing a transgenic animal according to any one of the preceding claims comprising (i) transfecting a target animal with a vector molecule according to claims 9 and 10.

11. A method of evaluating the carcinogenic potential of an agent or a composition in a specific tissue of an animal when applied intermittently comprising: (i) contacting the transgenic animal of any one of claims 1 to 8 with the agent or composition to be evaluated; (ii) blocking or inactivating expression of a gene of interest thus causing genetic aberrations within the cells of a specific tissue of the animal, (iii) identifying and determining said genetic aberrations; (iv) comparing the number of genetically altered cells in a sample from the treated animal with the number of genetically altered cells in a sample from an untreated transgenic animal or transgenic animal treated with a control agent, wherein the difference in the number of transformed cells in the treated animal, relative to the number of transformed cells in the absence of treatment or treatment with a control agent, indicates the carcinogenic potential of the test compound.

12. A method for evaluating the reversibility of the carcinogenic process induced by an agent or composition comprising: (i) contacting the transgenic animal of any one of claims 1 to 8 with the agent or composition to be evaluated intermittently according to a defined time schedule; (ii) blocking or inactivating expression of a gene of interest thus causing genetic aberrations within the cells of a specific tissue of the animal, (iii) identifying and determining said genetic aberrations; (iv) discontinuing contacting the transgenic animal with the agent or composition to be evaluated, comparing the number of genetically altered cells in a sample from the intermittently treated animal with the number of altered cells in a sample from a animal at a given time after treatment had been discontinued, and (iv) determining reversibility of the carcinogenic process.
